# EUROPEAN PATENT SPECIFICATION

(11) **EP 4 375 622 B1**
(45) Date of publication and mention of the grant of the patent: **18.06.2025**
(21) Application number: 23466004.1
(22) Date of filing: 22.11.2023
(51) Int. Cl.: G01F 1/66, G01N 21/05, G01N 21/27, G01N 21/25, G01N 21/3577, G01N 29/00, G01N 33/14, A47J 31/40, G01N 21/31

(54) **MULTIFUNCTIONAL DEVICE FOR MEASURING THE FLOW RATE OF LIQUIDS, IN PARTICULAR BEVERAGES**
MULTIFUNKTIONALE VORRICHTUNG ZUR MESSUNG DER DURCHFLUSSRATE VON FLÜSSIGKEITEN, INSBESONDERE GETRÄNKEN
DISPOSITIF MULTIFONCTIONNEL POUR MESURER LE DÉBIT DE LIQUIDES, EN PARTICULIER DE BOISSONS

(30) Priority: 23.11.2022 CZ 20220493
(43) Date of publication of application: 29.05.2024
(73) Proprietor: Smart Technology, s.r.o., 27371 Zlonice (CZ); Bc. Hedbávný Jakub, 27371 Zlonice (CZ)
(72) Inventor: Radim, Laga, 68801 Uherský Brod (CZ); Antonín, Stepán, 27401 Slaný (CZ)
(74) Representative: Vermirovsky, Tomas

(56) References cited:
- EP-A2- 0 145 384
- WO-A1-2007/148080
- WO-A1-2014/166734
- US-A1- 2007 227 263

## Description

### Field of the invention

The present invention relates to a device for measuring of flow rate of liquids, in particular beverages for a dispensing device, which enables the dispensed quantity of a beverage to be monitored and makes it possible to distinguish whether the beverage is flowing through the device or whether flushing with water or whether sanitation of the dispensing device is being carried out.

### Background of the invention

Either mechanical or ultrasonic flow meters are currently used in the dispensing devices for monitoring of dispensed quantity of the beverage. The mechanical flow meters operate based on sensing revolutions of a small propeller inside the flow meter and they operate with measurement deviation of about 57% at present. In addition, the mechanical flow meters cannot distinguish whether the beverage or flushing liquid or sanitation liquid is flowing through the flow meter, which adds to their inaccuracy. While the ultrasonic flow meters used in the industry provide higher accuracy, sensing flow rate with a 2-3% deviation, they also fail to detect the liquid flowing through the flow meter. EP0145384-A2 discloses a measuring apparatus arranged to measure the flow rate in a conduit of a fluid containing a particular gas component using ultrasonic transducers and the concentration of the particular gas component using light absorption of a light beam transmitted via a pair of windows in the wall of the conduit.

A multifunctional device for measuring of liquid flow rate according to the present invention solves all drawbacks mentioned above.

### Summary of the invention

According to the present invention, defined by claim 1, there is disclosed a multifunctional device for measuring liquid flow rate, which not only allows to accurately measure the volume of the fluid flowed through, but also allows to distinguish the type of the liquid flowed through, in particular to distinguish a beverage from a flushing fluid, and also allows to recognize the sanitization that has taken place, including the possible passage of a cleaning sponge or other means.

The subject matter of the present invention is the multifunctional device for measuring liquid flow rate, in particular a beverage, comprising an ultrasonic flow meter, wherein the multifunctional device further includes at least a white light emitting diode (i.e., the diode that emits white light), a sensor of red, green, blue, and white component of incident light, a microprocessor programmed to calibrate data output from the sensor of red, green, blue, and white light components depending on parameters of known liquids, which is connected to the white light emitting diode and the sensor of red, green, blue, and white light components, a memory unit including a definition table of red, green, blue, and white light components for reference liquids, wherein each reference liquid is defined by these colour components, wherein the microprocessor is further programmed to transmit the calibrated data from the sensor of red, green, blue, and white light components to a superordinate control and evaluation unit.

The microprocessor of the multifunctional device according to the present invention is particularly preferably programmed to collect data from the RGBW sensor and to carry out calibration based on individual reference fluids flowing in sequence, wherein it always receives information, e.g., via a data bus, which reference liquid flows through the flow meter. Data received from the RGBW sensor forms a quaternion of colour components Rx, Gx, Bx, and Wx for the medium in question, wherein x indicates type of the reference liquid. For each reference liquid x, the microprocessor particularly preferably calculates correction coefficients for each colour component. From the obtained set of quaternions of correction coefficients for individual media, the mean value is then calculated from the coefficients being calculated for each component, and the mean value is then used as a resulting correction coefficient of the component in question. The microprocessor then calculates the quaternion of coefficients, which it then multiplies by the measured data from the sensor of red, green, blue, and white light components to obtain values independent of deviations of specific elements used, in particular the white light emitting diode and the sensor of red, green, blue, and white light components. At the same time, the calibration removes the effect of ageing of these elements, or any other change thereof.

According to one preferred embodiment, the multifunctional device for measuring of liquid flow rate, in particular beverages, is further connected to the evaluation unit programmed to evaluate the type of liquid based on proportion of individual measured light components.

The multifunctional device for measuring of liquid flow rate, in particular beverages, according to the present invention, preferably includes the ultrasonic flow meter, which belongs to the group of speed flow meters evaluating the volumetric flow based on the measurement of the velocity of the flowing medium and knowledge of the flow cross-section. An ultrasonic signal spreading inside the flowing medium is used to measure speed. The ultrasonic flow meter includes an ultrasonic wave transmitter and an ultrasonic wave receiver. Particularly preferably, piezoelectric transducers are used as both transmitter and receiver, which may operate as both transmitter, i.e., a generator of the ultrasonic signal, and as receiver of the ultrasonic signal. The frequency of the ultrasonic waves used preferably ranges from 0.5 to 1MHz.

The ultrasonic flow meter of the multifunctional device according to the present invention comprises a measuring tube in which at least one pair of a transmitter and a receiver of an ultrasonic signal is embedded. This ultrasonic flow meter is particularly preferably arranged in a differential connection, in which the ultrasonic signal is transmitted both in the direction and against the direction of flow. The measurement of the flow rate is based on the idea of detecting the difference in the duration of the flight of an ultrasonic wave for a given distance through liquid in one direction (e.g., from the transmitter/receiver No. 1 to the transmitter/receiver No. 2) and the ultrasonic wave through the liquid over the same distance in the reverse direction (i.e., in this case, from the transmitter/receiver No. 2 to the transmitter/receiver No. 1). When the liquid is static in the device for measuring of liquid flow rate, especially beverages, the duration of the flight of the ultrasonic wave is the same in both directions. However, when the liquid starts to flow through the measuring tube of the device, the ultrasonic wave sent in the direction of the liquid flow is accelerated, whereas the ultrasonic wave sent in the direction opposite to the liquid flow is decelerated. The difference in these durations is multiplied by a constant derived from a combination of the measuring tube cross-section and the measured distance (e.g., the distance of two transmitters/receivers from each other) to give an approximate value of the actual flow rate. Higher measurement accuracy is achieved by performing a multipoint calibration. An advantage of the above- described differential connection of the ultrasonic flow meter of the multifunctional device according to the present invention is that it is not dependent on the specific conductivity or viscosity of the liquid medium.

As already mentioned above, the multifunctional device according to the present invention enables the recognition of the type of liquid. Hence, the multifunctional device for measuring of flow rate of liquids, in particular beverages, according to the invention, includes, for evaluating the type of liquid, a white light emitting diode (LED) with sufficient power to provide the necessary illumination of the liquid in the measuring tube of the ultrasonic flow meter. The sensor of red, green, blue, and white light components (hereinafter referred to as RGBW sensor only) detects the transmitted light divided into red, green, blue, and white components, wherein the light emitting diode as well as the RGBW sensor are connected to the microprocessor (the evaluation unit), connected to the memory unit containing an algorithm for calibrating the light captured by the RGBW sensor so as to provide a reliable determination of type of liquid passing through the multifunctional device according to the present invention at a given moment. The algorithm preferably operates with assumption that the white LED used may not always have the same luminous flux due to manufacturing tolerances, control circuit tolerances, and potential LED degradation over time, but the proportion of its colour spectrum components are assumed to be maintained. According to a particularly preferred embodiment of the multifunctional device according to the present invention, the individual components of the light captured by the RGBW sensor are further corrected according to the current temperature.

It is furthermore particularly preferable if the multifunctional device according to the present invention is further provided with an optical infrared gate comprising an infrared light emitting diode (hereinafter referred to as the IR diode) and an infrared light sensor (hereinafter referred to as the IR sensor) for detecting the passage of a sanitation sponge, wherein said optical infrared gate is connected to the microprocessor described above, or is configured to be connected to the control and evaluation unit provided with an algorithm for evaluating the passage of the sanitation sponge. The passage of infrared light from the IR diode to the IR sensor is temporarily interrupted by the passage of the sanitizing sponge, wherein the algorithm for evaluating the passage of the sanitation sponge is created to evaluate both the voltage drop across the IR sensor as an object passes between the IR diode and the IR sensor, the sanitation sponge providing better overlap than a possible air bubble in the liquid, and the overlap time. Particularly preferably, the algorithm for evaluating the passage of the sanitation sponge is created to evaluate whether the passing liquid is water or sanitation solution, so that it is obvious that the sanitizing is being performed correctly. Further, the algorithm is preferably created to evaluate whether the object overlying the sensor is sufficiently impervious, and whether the overlap time is within a defined range of the referenced time corresponds to the reference flow rate that is modified by current flow rate.

### Explanation of drawings

The invention will be explained by reference to the following examples of embodiments and to the accompanying drawings, in which:
Fig. 1 is exploded view of individual components of the multifunctional device according to the present invention,
Fig. 2 is top view of the assembled multifunctional device according to the present invention,
Fig. 3 is cross-section view of the multifunctional device according to the present invention,
Fig. 4 is view of the printed circuit board with the detectors,
Fig. 5 is view of the second printed circuit board with the light emitting diodes.

### Exemplary embodiments

The present invention will be described by reference to specific examples of embodiments and by reference to the accompanying drawings, but the invention is not intended to be limited to these described examples as these examples of embodiments are merely for the purpose of facilitating an understanding of the subject-matter of the present invention. Thus, the invention is limited only by the wording of the patent claims. Furthermore, it is important to note that the accompanying drawings are schematic only and are not intended as a detail description of the invention, and they should not be interpreted as limiting the scope of protection of the invention to the depicted embodiments. The drawings may exaggerate the size of certain members for illustrative purposes, and these may not be drawn to scale. The dimensions of individual members may not correspond to their actual dimensions when the invention is used.

The terms related to orientation in space, such as "left", "right", "bottom", "top", and the like, are herein used to help describe relations of one element or feature to another element(s) or feature(s) as show in the figures. The terms related to orientation in space may, however, include also other orientations of the element or feature in use or in operation in addition to the orientations illustrated in the figures. For example, when the device is rotated upside down in use in the figure, the elements described or illustrated as "top" or "above" the other elements or features in the figure will thus be oriented "under" other elements or features. Therefore, the exemplary term "top" may include both top and bottom orientation. However, the device may be also otherwise oriented (e.g., rotated by a certain angle with respect to a viewer or otherwise). Thus, it is important to note that the described terms related to orientation used herein must be then interpreted accordingly.

It should be noted that terms derived from the words "include" or "comprise" used in the description or claims should not be interpreted as limiting the scope to the terms further below mentioned, so as not to exclude the presence of other elements. Therefore, they should be interpreted as indicating the presented property, units, steps, or parts to which they refer, but presence or addition of one or more other properties, units, steps, or components, or groups thereof, is not ruled out. Therefore, the scope of a phrase "a device comprising components A and B" should not be limited to the device comprising only components A and B, but with respect to the present invention, it means that the components A and B are essential to the invention.

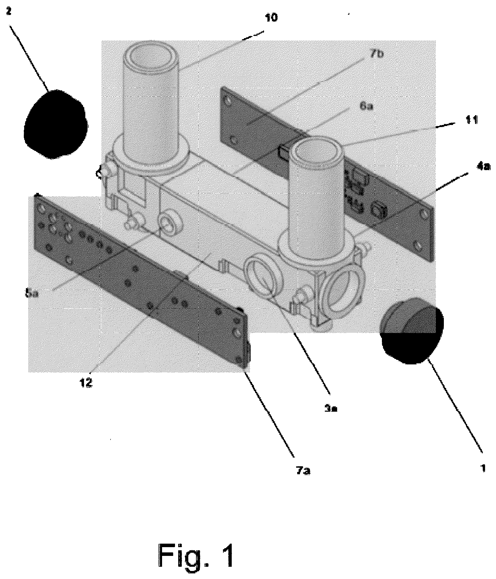

Fig. 1 to Fig. 3 illustrate a multifunctional device for measuring the flow rate of liquids, in particular beverages, according to the invention which includes a body 12 in which a measuring tube is formed, to which an input tube 10 and output tube 11 are fluidly connected from top to allow liquid to flow through the multifunctional device. Ultrasonic sensors 1 and 2 are arranged at the front opposite sides of the body 12 (left and right in Fig. 1) for measuring the flow rate in the measuring tube of the body 12.

According to the exemplary embodiment of the multifunctional device according to the present invention in Fig. 1 to 3, the body 12 has a square cross-section and the measuring tube through which the liquid flows is integrally made in the body 12 during its manufacture. The measuring tube may be made directly in the body 12, e.g., as a bore or a cast cavity, or may be made separately and inserted into the body 12 in a suitable manner.

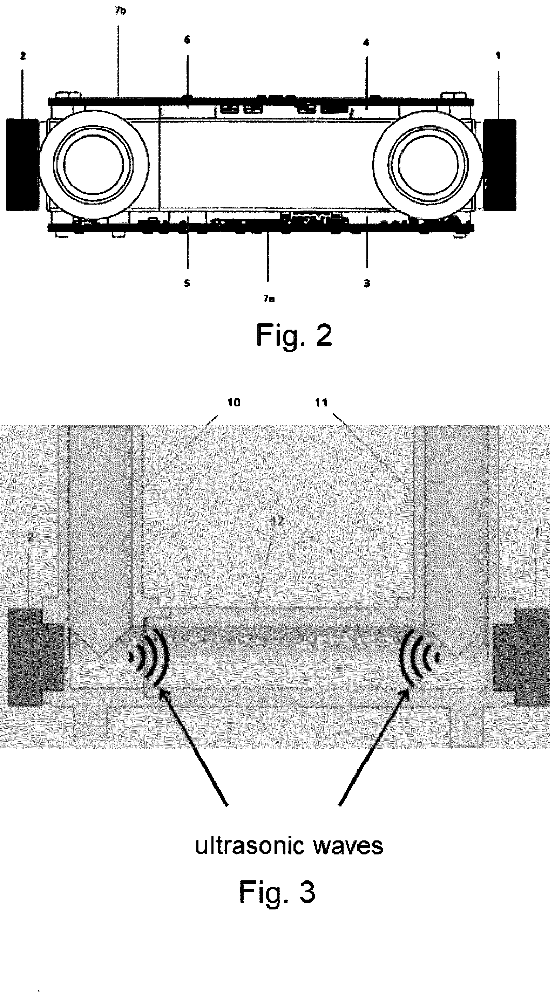

Fig. 1 illustrates the exploded view of the multifunctional device for measuring of flow of liquids, in particular beverages, according to the present invention, wherein the body 12 with the measuring tube is provided on its upper side with the input tube 10 and output tube 11 for connecting the multifunctional device to a measured dispensing circuit of the beverage or other liquid in question, and has formed thereon a white light-emitting diode mounting assembly 6a, an RGBW sensor mounting assembly 5a, an IR sensor mounting assembly 3a and an mounting assembly 4a for IR diode 3.Further, first ultrasonic wave transmitter/receiver 1 and an opposing second ultrasonic wave transmitter/receiver 2 can be seen in Fig 1. In Fig. 2, it can be seen that the white light emitting diode 6, i.e., the diode that emits white light, is arranged on one lateral longitudinal side of the body 12, , while on the opposite lateral longitudinal side of the body 12, a sensor 5 for sensing the red, green, blue and white light components, also referred to as an RGBW sensor, is arranged in the RGBW sensor mounting assembly 5a, wherein mutual arrangements of the RGBW sensor 5 and the white light emitting diode 6 ensure that the light from the white light emitting diode 6 is incident on the RGBW sensor 5. Since the whole body 12 in this exemplary embodiment of the multifunctional device according to the present invention is made of a translucent material, i.e., including the measuring tube, the light sent from the light emitting diode 6 to the RGBW sensor 5 is allowed to pass through the body 12 and thus through the liquid in the measuring tube of the body 12. Alternatively, the whole or at least a part of the body 12 may be made from a non-translucent material, wherein the body 12 is provided with a translucent portion arranged to allow the passage of light from the white light emitting diode 6 to the RGBW sensor 5 at the location at which it is necessary to allow the passage of light from the white light emitting diode 6 to the RGBW sensor 5. This translucent portion of the body 12 may be formed as translucent windows at least at appropriate positions in both opposite walls of the body 12 or as an inserted translucent segment of the body 12 in a given area, and the like. If the liquid to be measured is a beverage at least the measuring tube in the body 12 or the whole body 12, and the input tube 10 and output tube 11 for guiding of the liquid through the multifunctional device according to the present invention are made from a material certified for contact with foodstuffs.. As a result of the design of the multifunctional device according to the present invention, there is no contact of the liquid with the measuring elements and therefore no potential contamination thereof. In this exemplary embodiment, the internal diameter of the measuring tube has been selected particularly preferably to be 9.5mm, with respect to optimum passage of the sanitation sponge. However, it will be apparent to one skilled in the art that other diameters of the measuring tube body 12 are suitable. For simplicity, the embodiment in Fig. 1 to 3 includes also a cleaning sponge passage detector comprising an infrared light emitting diode 4 (IR diode) and an opposite infrared light sensor 3, which are arranged on the body 12 in a way similar to the above described alternative embodiments of the body 12 in association with the white light emitting diode 6 and the RGBW sensor 5, that is to say, the IR diode 4 is arranged in a mounting assembly 4a formed on the body 12, while the infrared light sensor 3 is arranged in a mounting assembly 3a, wherein the IR diode 4 illuminates the space in the measuring tube of the body 12 by infrared light, so that the flux of infrared light to the infrared the light sensor 3 is interrupted or reduced when the cleaning sponge passes through the measuring tube. Also in this example, at least the region of the body 12 and the measuring tube through which the infrared light from the IR diode 4 is to pass is translucent, similarly to the above-described passage of white light from the white light-emitting diode 6 through the body 12 and the measuring tube, i.e., at least a portion of both the body 12 and the measuring tube at the location of arrangement of the IR diode 4 and the location of the IR sensor 3 is translucent. However, according to further not shown embodiment of the multifunctional device according to the present invention, this device does not include the described cleaning sponge passage detector. The ultrasonic wave transmitters/receivers 1, 2 are piezoelectric transducers with a wave frequency of 1MHz, which are capable of serving as both transmitter and receiver of the ultrasonic waves.

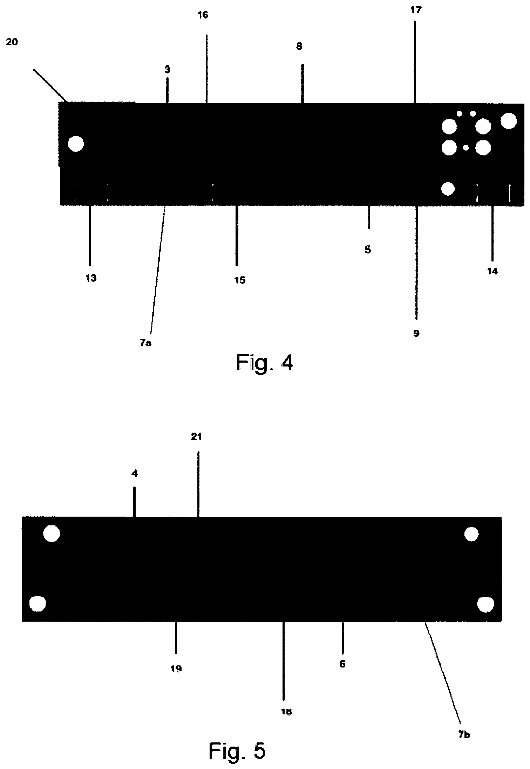

In Fig. 1 to 3, the multifunctional device according to the present invention is provided along its longitudinal sides with two printed circuit boards 7a, 7b connected by a flat cable (not shown). The white light emitting diode 6 and the IR diode 4 are arranged on one printed circuit board 7b (see Fig. 5), and the microprocessor 8, the memory unit 9 for storing of a programme with a respective algorithm or programmes with additional algorithms, the RGBW sensor 5 and the IR sensor 3, a power supply circuit 20 and other service components are arranged on the other printed circuit board 7a (see Fig. 4). It will be obvious to one skilled in the art that combined arrangement of the microprocessor 8 and the memory unit 9 on the printed circuit board 7a with both sensors, i.e., the IR sensor 3 and the RGBW sensor 5, located thereon, is only preferable but not the only possible arrangement, and that other arrangements are possible. According to further not shown exemplary embodiments, the multifunctional device according to the present invention is provided with a plurality of printed circuit boards placed, for example, on the top or bottom side or other sides of the multifunctional device, or spaced apart from another printed circuit board, or optionally, for example, the side board may be divided into two boards arranged side by side, etc. Thus, it is also envisaged to arrange the multifunctional device according to the invention with a plurality of boards on one side of the body 12 connected only by cables, etc. However, it is possible to use light emitting diodes and sensors which are not placed on the printed circuit boards but are attached directly to the body of the ultrasonic detector. Thus, many different arrangements of individual components and circuit boards are possible.

Fig. 2 then illustrates top view of the assembled multifunctional device according to the present invention, which already has the white light emitting diode 6 and the IR diode 4 arranged on one printed circuit board 7b, the RGBW sensor 5 and the IR sensor 3 arranged on the other printed circuit board 7a, as well as both ultrasonic wave transmitters/receivers 1, 2 arranged in operation position. The printed circuit board 7a with both sensors 3, 5 is particularly preferably provided with a temperature sensor 17 for correcting the measured values of the RGBW components, see Fig. 4.

Figure 3 then illustrates cross-section of the multifunctional device according to the present invention along a vertical plane of the device guided by its centre axis. In particular, the hydraulic connection of the measuring tube of the body 12 with the input tube 10 and output tube 11 is apparent in this figure, while the radiated ultrasonic waves are also shown schematically.

In Fig. 4 is then illustrated an exemplary embodiment of the printed circuit board 7a, on which there is the IR sensor 3, the RGBW sensor 5, the microprocessor 8, the memory unit 9, there is also a connector 14 for connecting the second transmitter/receiver 2, a connector 13 for connecting the first transmitter/receiver 1, the temperature sensor 17, a data connector 15 for transferring data from the microprocessor 8 to a superordinate evaluation unit, an interconnection connector 16 for connecting to the second printed circuit board 7b, and other service components such as a power supply 20, etc..

Fig. 5 then illustrates the printed circuit board 7b with the IR diode 4 and the white light emitting diode 6, power sources 18 and 21 for powering the IR diode 4 and the white light emitting diode 6, the interconnection connector 19 for interconnecting with the first printed circuit board 7a, etc. It is important to note that the above-described exemplary embodiments are not intended to be limit the scope of protection to the specific embodiments described herein and are intended primary to aid in understanding the subject matter of the present invention. The present invention should be understood to be limited only by the wording claims.

### Industrial applicability

The practical use of the proposed solution is considered particularly preferably for the measurement and recognition of beverages dispensed by the dispensing device with the possibility to monitor in particular the implementation of required inspections and sanitation. However, the proposed solution may be used wherever it is necessary to monitor not only quantity of flowed liquid but also a type of the flowed liquid, etc.

## Claims

1. A multifunctional device for measuring the flow rate of a liquid, in particular beverage, comprising an ultrasonic flow meter comprising a body (12) with a measuring tube for passing the measured liquid, which is provided with an ultrasonic transmitter and receiver (1, 2) at each of the two opposite front ends of the measuring tube, **characterized in that**
it further comprises at least a white light emitting diode (6), a sensor (5) of red, green, blue, and white light components, a microprocessor, and a memory unit comprising a definition table of the red, green, blue, and white light components of reference liquids, wherein each reference liquid is defined by these colour components,
said white light emitting diode (6) is arranged on one side of the measuring tube of the flow meter, while the sensor (5) of red, green, blue, and white light components is arranged on the opposite side of the measuring tube, wherein the body (12) with the measuring tube is translucent at least in its portion to allow light to pass from the white light emitting diode (6) to the sensor (5) of red, green, blue, and white light components,
wherein the microprocessor is connected to the white light emitting diode (6) and the sensor (5) of red, green, blue, and white light components, and is programmed to calibrate data output from the sensor (5) of red, green, blue, and white light components depending on parameters of known reference liquids, and furthermore, this microprocessor is programmed to transmit the calibrated data from the sensor (5) of red, green, blue, and white light components to a superordinate evaluation unit.

2. The multifunctional device according to claim 1 **characterized in that** it further includes an infrared light emitting diode (4) and a sensor (3) of infrared radiation for detecting the passage of a sanitation sponge, wherein the infrared light emitting diode (6) is arranged on one side of the measuring tube of the flow meter, whereas the sensor (5) of the infrared light is arranged on the opposite side of the measuring tube, wherein the body (12) with the measuring tube is also translucent in this portion to allow infrared light to pass from the infrared light emitting diode (6) to the sensor (5) of the infrared light.

3. The multifunctional device according to claim or 2 **characterized in that** the whole body (12) is translucent.

4. The multifunctional device according to any one of claims 1 to 3 **characterized in that** it further includes a temperature sensor, wherein the microprocessor is further programmed to correct the individual components of the light captured by the sensor (5) of red, green, blue, and white light components according to actual temperature measured by the temperature sensor.

## Patentansprüche

1. Eine multifunktionale Vorrichtung zum Messen des Durchflusses einer Flüssigkeit, insbesondere eines Getränks, mit einem Ultraschall-Durchflussmesser, der ein Gehäuse (12) mit einem Messrohr zum Durchleiten der zu messenden Flüssigkeit umfasst, der mit einem Ultraschall-Sender und - Empfänger (1, 2) an jedem der beiden gegenüberliegenden Enden des Messrohrs versehen ist,
**dadurch gekennzeichnet, dass**
- weiter mindestens eine Weißlicht-Emissionsdiode (6), ein Sensor (5) für die roten, grünen, blauen und weißen Lichtkomponenten, ein Mikroprozessor und ein Speicher mit einer Definitionstabelle der roten, grünen, blauen und weißen Lichtkomponenten von Referenzflüssigkeiten beinhaltet ist, wobei jede Referenzflüssigkeit durch diese Farbkomponenten definiert ist,
- die weiße Leuchtdiode (6) auf einer Seite des Messrohrs des Durchflussmessers angeordnet ist, während ein Sensor (5) der roten, grünen, blauen und weißen Lichtkomponenten auf der gegenüberliegenden Seite des Messrohrs angeordnet ist, wobei das Gehäuse (12) mit dem Messrohr zumindest zu einem Teil lichtdurchlässig ist, um zu ermöglichen, dass Licht von der weißen Leuchtdiode (6) zu dem Sensor (5) der roten, grünen, blauen und weißen Lichtkomponenten gelangt,
- der Mikroprozessor mit der Weißlicht emittierenden Diode (6) und dem Sensor (5) der roten, grünen, blauen und weißen Lichtkomponente gekoppelt ist und so programmiert ist, dass er die vom Sensor (5) der roten, grünen, blauen und weißen Lichtkomponenten ausgegebenen Daten relativ zu den Parametern der Referenzflüssigkeiten kalibriert, der Mikroprozessor ist ferner programmiert, die kalibrierten Daten des Sensors (5) für die roten, grünen, blauen und weißen Lichtkomponenten an eine übergeordnete Auswerteeinheit zu senden.

2. Multifunktionsgerät nach Anspruch 1, **dadurch gekennzeichnet, dass** es eine Infrarot-Leuchtdiode (4) und einen Infrarotsensor (3) zur Erfassung des Durchgangs eines Sanitärschwamms aufweist, wobei die Infrarot-Leuchtdiode (6) auf einer Seite des Messrohrs des Durchflussmessers angeordnet ist, während der Infrarot-Lichtsensor (5) auf der gegenüberliegenden Seite des Messrohrs angeordnet ist, wobei das Gehäuse (12) mit dem Messrohr auch in diesem Teil lichtdurchlässig ist, um Infrarotlicht von der Infrarot-Licht emittierenden Diode (6) zu dem Infrarot-Lichtsensor (5) durchzulassen.

3. Multifunktionsgerät nach Anspruch 1 oder 2, **dadurch gekennzeichnet, dass** das gesamte Gehäuse (12) lichtdurchlässig ist.

4. Multifunktionsgerät nach einem der Ansprüche 1 bis 3, **dadurch gekennzeichnet, dass** es weiter einen Temperatursensor umfasst, wobei der Mikroprozessor so programmiert ist, dass er die einzelnen Komponenten des vom Sensor (5) erfassten Lichts der roten, grünen, blauen und weißen Komponenten des Lichts entsprechend der vom Temperatursensor gemessenen tatsächlichen Temperatur korrigiert.

## Revendications

1. Dispositif multifonctionnel de mesure du débit d'un liquide, notamment d'une boisson, comportant un débitmètre à ultrasons comprenant un corps (12) avec le tube de mesure pour le passage du liquide mesuré, le corps étant muni à chacune des deux extrémités opposées du tube de mesure d'un émetteur et d'un récepteur à ultrasons (1, 2), **caractérisé en ce que**
- il comporte en outre au moins une diode électroluminescente blanche (6), un capteur (5) de composantes rouge, verte, bleue et blanche de la lumière, un microprocesseur et une unité de mémoire contenant une table de définition des composantes rouge, verte, bleue et blanche de la lumière pour des liquides de référence, chaque liquide de référence étant défini par ces composantes de couleur,
- ladite diode électroluminescente blanche (6) est disposée d'un côté du tube de mesure du débitmètre, tandis que le capteur (5) de composantes rouge, verte, bleue et blanche de la lumière est disposé du côté opposé du tube de mesure, le corps (12) avec le tube de mesure étant translucide au moins dans sa partie destinée à permettre le passage de la lumière provenant de la diode électroluminescente blanche (6) vers le capteur (5) de composantes rouge, verte, bleue et blanche de la lumière,
- le microprocesseur est connecté à la diode électroluminescente blanche (6) et au capteur (5) de composantes rouge, verte, bleue et blanche de la lumière, et il est programmé pour calibrer les données de sortie du capteur (5) de composantes rouge, verte, bleue et blanche de la lumière en fonction des paramètres de liquides de référence connus, et en outre, ce microprocesseur est programmé pour envoyer les données calibrées provenant du capteur (5) de composantes rouge, verte, bleue et blanche de la lumière vers l'unité d'évaluation supérieure.

2. Dispositif multifonctionnel selon la revendication 1, **caractérisé en ce qu'**il comporte en outre une diode électroluminescente infrarouge (4) et un capteur (3) de rayonnement infrarouge pour détecter le passage de l'éponge de sanitation, la diode électroluminescente infrarouge (6) étant disposée d'un côté du tube de mesure du débitmètre, tandis que le capteur (5) de lumière infrarouge est disposé du côté opposé du tube de mesure, le corps (12) avec le tube de mesure étant translucide également dans cette partie pour permettre le passage de la lumière infrarouge provenant de la diode électroluminescente infrarouge (6) vers le capteur (5) de lumière infrarouge.

3. Dispositif multifonctionnel selon la revendication 1 ou 2, **caractérisé en ce que** le corps (12) entier est translucide.

4. Dispositif multifonctionnel selon l'une quelconque des revendications 1 à 3, **caractérisé en ce qu'**il comporte en outre un capteur de température, le microprocesseur étant en outre programmé pour corriger les composantes individuelles de la lumière captée par le capteur (5) de composantes rouge, verte, bleue et blanche de la lumière en fonction de la température courante mesurée par ce capteur de température.
